# EUROPEAN PATENT APPLICATION

(11) **EP 1 977 765 A1**
(43) Date of publication of application: **08.10.2008**
(21) Application number: 07300920.1
(22) Date of filing: 03.04.2007
(51) Int. Cl.: A61K 47/48, A61P 35/00

(54) **Peptide prodrugs**

(71) Applicant: Diatos, 75014 Paris (FR)
(72) Inventor: Matthieu, Michel, F-78100 Le Vesinet (FR); Dubois, Vincent, F-91190 Gif-sur-Yvette (FR); Tranchant, Isabelle, F-94270 Le Kremlin-Bicetre (FR); Kearsey, Jonathan, F-78125 Hermeray (FR)
(74) Representative: Bredema

(57) **Abstract**

The present invention relates to the field of prodrugs, and more particularly prodrugs that arc intended for the treatment and/or diagnosis of tumors and/or inflammatory reactions.

SEQUENCE LISTING

<110>  DIATOS

<120>  PEPTIDE PRODRUGS

<130>  B0568EP

<160>  7

<170>  PatentIn version 3.3

<210>  1
<211>  4
<212>  PRT
<213>  artificial

<220>
<223>  cleavable oligopeptide

<400>  1

Ala Leu Ala Leu
1


<210>  2
<211>  4
<212>  PRT
<213>  artificial

<220>
<223>  cleavable oligopeptide


<220>
<221>  MOD_RES
<222>  (1)..(1)
<223>  bAla

<400>  2

Ala Leu Ala Leu
1


<210>  3
<211>  5
<212>  PRT
<213>  artificial

<220>
<223>  cleavable oligopeptide

<400>  3

Ala Leu Lys Leu Leu
1               5


<210>  4
<211>  6
<212>  PRT
<213>  artificial

<220>
<223>  cleavable oligopeptide

<400>  4

Ala Tyr Gly Gly Phe Leu
1               5


<210>  5
<211>  7
<212>  PRT
<213>  artificial

<220>
<223>  cleavable oligopeptide

<400>  5

His Ser Ser Lys Leu Gln Leu
1               5


<210>  6
<211>  8
<212>  PRT
<213>  artificial

<220>
<223>  cleavable oligopeptide

<400>  6

Gly Pro Leu Gly Ile Ala Gly Gln
1               5


<210>  7
<211>  9
<212>  PRT
<213>  artificial

<220>
<223>  cleavable oligopeptide

<400>  7

Cys Asp Cys Arg Gly Asp Cys Phe Cys
1               5

## Description

### BACKGROUND OF THE INVENTION

The present invention relates to the field of prodrugs, and more particularly prodrugs that are intended for the treatment and/or diagnosis of tumors and/or inflammatory reactions.

Prodrugs are pharmacologically inactive molecules that are transformed *in vivo* into active therapeutic agents after certain chemical or enzymatic modifications of their structure. Prodrugs are intended to release the therapeutic agent at a target site or tissue rather than in the circulatory structure or in a non-target tissue. They also make it possible to increase *in vivo* the therapeutic index of therapeutic agents such as anti-tumor agents like anthracyclines (such as doxorubicin) and vinca alkaloids or anti-tumor agents that have anti-inflammatory effects like methotrexate. Thus, several prodrugs have been developed to date for the purpose of obtaining high action specificity, a reduced toxicity and an improved stability in the blood and/or the plasma.

Prodrugs comprising a stabilizing or masking group covalently linked to a therapeutic agent via an oligopeptide cleavable by an enzyme, specifically by a peptidase, have been described in the prior art. See, for example, U.S. Pat No US 4,703,107 and US 5,618,790.

International patent application No. WO 96/05863 describes peptide prodrugs of therapeutic agents, selectively cleavable by factors secreted by target cells so as to enable the therapeutic agent to penetrate into said target cells. Such prodrugs have a stabilizing group, for example a beta-alanyl or succinyl moiety, ensuring that the prodrug is stable in serum and circulating blood. *In vivo* toxicity and activity studies with the prodrug beta-alanyl-leucyl-alanyl-leucyl-doxorubicin according to WO 96/05863 showed a reduction in toxicity and a more important inhibition of tumor growth compared to doxorubicin (Dubois et al., 2002, Cancer Res., 62(8):2327-31, 2002).

International patent application No. WO 00/33888 discloses the addition of a group that masks the positive charge of beta-alanine to the prodrug beta-alanyl-leucyl-alanyl-leucyl-doxorubicin described above so as to reduce its toxicity. This masking group can be, for example, a succinic acid.

Masking groups have also been described in PCT application No. WO 00/71571 aiming to increase the hydrophilicity of peptide prodrugs and thus enhance their solubility in aqueous media. For example, disclosed but not exemplified masking groups are cyclic hydrocarbon radicals having 3 to 8 carbon atoms which may optionally be substituted by one or several hydroxyl, amino, C₁-C₆-alkyl-amino, di- C₁-C₆-alkyl-amino, C₁-C₆-alkyl, C₁-C₆-alkyloxy, thiol, C₁-C₆-alkyl-thio, =O, =NH, -CHO, -COOH, - COOCH3, -COOCH₂CH₃, -CONH₂, -NHC(=NH)NH₂, or halogen substituents, which may be identical to one another or different.

The present invention aims to provide peptide prodrugs of a therapeutic agent or marker, which improve solubility of the therapeutic agent or marker, reduce its toxic or non-desirable side-effects, improve its therapeutic index, improve its toxicological profile and/or modify its tissue distribution and/or metabolism, as compared to known peptide prodrugs.

### BRIEF SUMMARY OF THE INVENTION

The present invention relates to the modification of a peptide prodrug with a 1,2,3,4-cyclobutanetetracarboxylic acid (hereinafter designated by TA) derived moiety for enhancing the effects of said peptide prodrug. The present invention further discloses novel peptide prodrugs presenting said TA derived moiety, compositions comprising these prodrugs and uses thereof.

According to a first aspect, the invention relates to a compound (peptide prodrug) comprising a therapeutic agent linked directly or indirectly to a cleavable oligopeptide which, in turn, is linked directly or indirectly to a TA derived moiety. The compounds of the present invention provide numerous benefits including, in particular, improvement of the therapeutic index and of the solubility of the therapeutic agent.

The invention also relates to pharmaceutical compositions comprising such peptide prodrugs and therapeutic uses thereof, in particular for the treatment of various types of diseases, including cancers.

The invention also relates to the use of at least one compound defined herein below for the preparation of a medicament for treating a number of disorders.

### DETAILED DESCRIPTION OF THE INVENTION

In one aspect, the invention provides a compound comprising a therapeutic agent or marker linked directly or indirectly to an oligopeptide which, in turn, is linked directly or indirectly to a 1,2,3,4-cyclobutanetetracarboxylic acid derived moiety.

1,2,3,4-cyclobutanetetracarboxylic acid (TA) is a well-known and commercially available compound. It has the following formula, C₈H₈O₈, or formula (I):

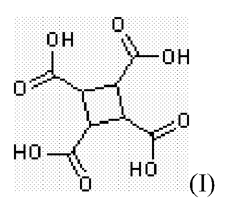

The present inventors have shown that modification of a peptide prodrug with TA leads to several beneficial advantages over the peptide prodrugs of the prior art, including the reduction of the toxicity of the peptide prodrug of the invention compared to the free drug and the increase of its efficacy.

Accordingly, it is an aspect of the invention to provide peptide prodrugs which are compounds of formula (II) 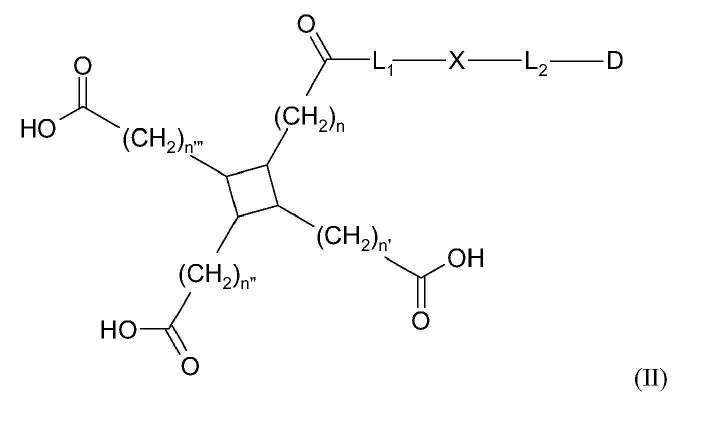 wherein
L₁ and L₂ are independently a covalent bond or a linking moiety,
n, n', n" and n"' independently represent an integer comprised between 0 and 10,
X is a cleavable oligopeptide moiety, and
D is a therapeutic agent or marker
or the pharmaceutically acceptable salts thereof.

Advantageously, modification of a therapeutic agent such as to obtain a compound according to the invention can reduce some of the side effects of said therapeutic agent. Furthermore, peptide prodrugs of the invention present enhanced stability/efficiency and reduced toxicity of the therapeutic agent compared to known peptide prodrugs of the prior art.

Advantageously, the TA derived moiety in the compounds of formula (II) blocks the cleavage of the compounds of the invention by enzymes (e.g. peptidases, in particular exopeptidases) *in vivo* in the blood (or the circulatory structure) or in the extracellular space of non-target tissue. The present inventors have demonstrated that addition of a TA derived moiety results in an enhancement of the therapeutic efficiency compared to known peptide prodrugs and a reduction of the toxicity of the prodrug in comparison with the free drug. Additionally, this TA derived moiety likely provides preferable charge and/or other physical characteristics to the compounds of the invention. It also improves the pharmacokinetic properties of the prodrug compared to that observed with the prodrugs in the prior art. This result is obtained in particular when the renal elimination of the compounds of the invention or when the degradation or biotransformation by the hepatic metabolism of the compounds of the invention are reduced.

Addition of a TA derived moiety to a peptide prodrug according to the present invention thus provides a potent way to enhance the beneficial effects of said peptide prodrug.

In the compounds according to the invention, n, n', n" and n"' independently represent an integer comprised between 0 and 10, preferably comprised between 0 and 5 (i.e. n, n', n" and n"' independently represent 0, 1, 2, 3, 4 or 5). In a particular embodiment of the invention, in the following formula (III), n=n'=n"=n"'=0 :

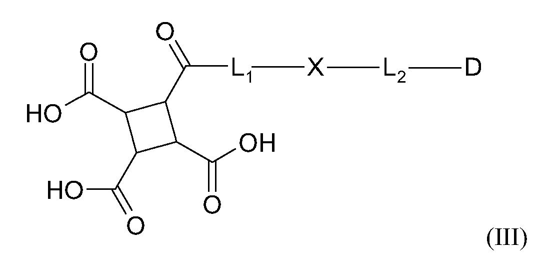

The compounds of the invention comprise a cleavable oligopeptide moiety directly or indirectly linked to the TA derived moiety. The cleavage of the oligopeptide moiety allows for activation of the therapeutic agent or marker by releasing the agent or marker from the remaining part of the prodrug in a specific location, *i*.*e*. in the environment of a target tissue or cells.

According to the present invention, a cleavable oligopeptide is an oligopeptide that can be cleaved selectively by a target tissue- or cell- associated enzyme (or peptidase), i.e. an oligopeptide which can be cleaved by an enzyme that is present in the environment of the target cells and which is only slightly, and preferably not at all, degraded in the circulatory structure or close to non-target cells. The expression "in the environment of the target cells" means that the enzyme is present close to or on the target cells.

It should be noted that even if the cleavage is not carried out on or only close to the target cells, the fact that the cleavage is carried out preferentially (or in large part or in the majority of the cases) close to or on the target cells makes this cleavage selective. For example, the cleavage is qualified selective when the enzyme is in a larger concentration close to or at the target cells relative to other parts of the organism.

The amino acid sequence of the cleavable oligopeptide moiety of the invention is selected for susceptibility to cleavage by at least one enzyme which is present in the environment of the target cells and which is only slightly, and preferably not at all, degraded in the circulatory structure or close to non-target cells.

"Target cells" are defined more particularly as cells that are involved in a pathological condition that are preferred targets for therapeutic or diagnostic activity. "Target tissue" comprises target cells themselves (e.g., tumor cells) but also any cell in the environment of the target cells, such as endothelial and stromal cells. These target cells are preferably selected from the following non limiting group: primary or secondary (metastases) tumor cells, stromal cells of the environment of primary or secondary tumors, neoangiogenic endothelial cells of tumors, macrophages, monocytes, polymorphonuclear leukocytes, lymphocytes, polynuclear cells infiltrating the tumors and the tumor metastases and cells participating in inflammatory reactions, especially associated with rheumatic diseases (e.g. macrophages, neutrophils, osteoblasts, osteocytes and monocytes).

The sequence of the cleavable oligopeptide moiety of the compounds of the invention is selected in accordance to the specific enzymes present in the environment of the target cells or tissue to treat with the therapeutic agent or to monitor with the marker comprised of the said compound of the invention. Indeed, as specified above, the cleavable oligopeptide moiety of the compounds of the invention are characterized by their susceptibility to cleavage by such target tissue- or target cell-associated enzymes.

A "target tissue- or cell-associated enzyme" is defined as a membrane enzyme or an enzyme that is released by the target cells in the extracellular medium surrounding these target cells. Target tissue-associated enzymes according to the invention are preferably peptidases, endopeptidases and lysosomal enzymes (enzymes located within lysosomes). According to a preferred embodiment of the invention, the enzyme is a specific peptidase of target tumor cells, stromal cells of the environment of the target tumor, neoangiogenic endothelial cells, macrophages or monocytes. In one embodiment of the invention, when the target tissue is a tumor, the enzyme can be selected from neprilysin (CALLA or CD10), thimet oligopeptidase (TOP), prostate specific antigen (PSA), plasmin, legumain, collagenases, urokinase, cathepsins, human fibroblast activation protein (FAPα) and the matrix metallopeptidases (these enzymes being well known for one skilled in the art). More preferred enzymes associated with target cells are neprilysin (CALLA or CD10) or thimet oligopeptidase (TOP).

The term "oligopeptide" is used herein to refer to a polymer of amino acid residues. The term refers to polymers of naturally occurring amino acids as well as to polymers of non naturally occurring amino acids or to polymers in which one or more amino acid residue is an artificial chemical mimetic of a corresponding naturally occurring amino acid.

The term "amino acid" refers to naturally occurring and non-natural amino acids, as well as amino acid analogs and amino acid mimetics that function in a manner similar to the naturally occurring amino acids.

"Naturally occurring amino acids" are those amino acids encoded by the genetic code as well as those amino acids that are not encoded by the genetic code and/or are later modified, e.g., beta-alanine (β-alanine), D-serine, hydroxyproline, γ-carboxyglutamate, and O-phosphoserine.

"Amino acid analogs" have the same basic chemical structure as naturally occurring amino acids, i.e., hydrocarbon chain comprising an amino group and a carboxyl group, some of the carbons being substituted or not, e.g., homoserine, norleucine, methionine sulfoxide, methionine methyl sulfonium. Such analogs have modified lateral chains (e.g., norleucine) or modified backbones, but retain the same basic chemical structure as a naturally occurring amino acid (e.g. beta amino acids, amino acids in D conformation).

"Amino acid mimetics" have a structure that is different from the general chemical structure of an amino acid, but that functions in a manner similar to a naturally occurring amino acid, e.g. benzamidine arginine, azetidine 2-carboxylic acid, D-cyclohexylglycine.

The referenced amino acids are represented in this invention either in three-letter code or in one-letter code, these codes are well known to one skilled in the art.

The cleavable oligopeptide moiety of the compounds of the invention preferably comprises, or alternatively consists of, between 2 and 10 amino acids, and more preferably between 3 to 7 amino acids.

In some embodiments of the invention, the cleavable oligopeptide comprises, or alternatively consists of, one or more of the following amino acid sequences (preferably in conformation L): Arg-Leu, Arg-Phe, Arg-Val, Ala-Phe, Ala-Leu, Ala-Tyr, Cys-Arg, Cys-Asp, Cys-Phe, Gln-Phe, Gly-Asp, Gly-Phe, Gly-Leu, Gly-Gln, Gly-Gly, Gly-Pro, His-Ser, Ile-Ala, Leu-Ala, Leu-Gln, Leu-Gly, Leu-Leu, Leu-Phe, Leu-Tyr, Lys-Leu, Met-Leu, Pro-Phe, Pro-Tyr, Pro-Leu, Phe-Leu, Phe-Phe, Tyr-Ile, Tyr-Pro, Tyr-Leu, Val-Tyr, Val-Phe, Ser-Leu and Ser-Lys.

In a preferred embodiment of the invention, the oligopeptide comprises, or alternatively consists of, one or more of the following sequences (preferably in conformation L): (Leu)y-(Ala-Leu)x-Ala-Leu or (Leu)y-(Ala-Leu)x-Ala-Phe wherein Leu is leucine, Ala is alanine, Phe is phenylalanine, y = 0 or 1 and x = 1, 2, or 3.

In another preferred embodiment of the invention, the oligopeptide comprises, or alternatively consists of, one or more of the following sequences (preferably in conformation L): Ala-Phe-Lys, Ala-Leu-Ala-Leu (SEQ ID NO:1) or beta-Ala-Leu-Ala-Leu (SEQ ID NO:2), Ala-Leu-Lys-Leu-Leu (SEQ ID NO:3), Ala-Tyr-Gly-Gly-Phe-Leu (SEQ ID NO:4), His-Ser-Ser-Lys-Leu-Gln-Leu (SEQ ID NO:5), Gly-Pro-Leu-Gly-Ile-Ala-Gly-Gln (SEQ ID NO:6) and Cys-Asp-Cys-Arg-Gly-Asp-Cys-Phe-Cys (SEQ ID NO:7).

In a particular embodiment of the invention, compounds of formula (II) are those wherein X comprises, or consists of, the sequence of SEQ ID NO: 1: Ala-Leu-Ala-Leu. This amino acid sequence is cleaved in particular by neprilysin, thimet oligopeptidase or cathepsin B enzymes.

One skilled in the art knows other amino acid sequences that can be cleaved selectively by a specific enzyme of tumor cells, such as those described in WO 00/33888 filed by COULTER PHARMACEUTICAL (see amino acid sequences described pages 13 to 15), WO 01/68145 filed by DUPONT PHARMACEUTICALS (see amino acid sequences described SEQ ID Nos 1 to 210), WO 01/95943 filed by CORIXA (see amino acid sequences described pages 16 to 18), WO 02/00263 filed by CORIXA (see amino acid sequences described table 1, page 16), WO 02/100353 filed by MEDAREX (see amino acid sequences described SEQ ID Nos 1 to 36), WO 02/07770 filed by PHARMACIA & UPJOHN (see amino acid sequences described page 3), WO 99/28345 filed by MERCK & CO (see amino acid sequences described SEQ ID Nos 1 to 125), or WO 03/094972 filed by BOEHRINGER INGELHEIM PHARMA, each incorporated herein by reference in their entirety and for all purposes.

Furthermore, one skilled in the art can refer to Handbook of Proteolytic Enzymes (A.J. Barrett, N.D. Rawlings, and J.F. Woessner eds. Academic Press, 1998; incorporated herein by reference in its entirety and for all purposes) to characterize the substrate-specificity of enzymes.

For the compounds of the invention to be effective, they typically undergo *in vivo* modification releasing the active therapeutic agent or an active derivative of the therapeutic agent which is able to exert its biological activity on the target cell either after entry into the target cell or without entry (e.g., if the therapeutic agent is a ligand of a membrane receptor like tumor necrosis factors). In one embodiment of the invention, an initial cleavage within the cleavable oligopeptide portion of the compound of the invention may result in a fragment of the compound that is competent for transport into the target cell. Alternatively, further cleavage by one or more peptidases may be required to result in a transport-competent fragment of the compound. The active or transport-competent fragment of the compound comprises at least the therapeutic agent and a part of the compound of formula (II) that can enter into or interact with the target cells to exert a therapeutic effect directly or upon further conversion within the target cell.

The target cells-associated enzymes defined above are able to selectively cleave the cleavable oligopeptide moiety so as to make possible the release of the therapeutic agent (or marker) or the release of the therapeutic agent (or marker) linked to a portion of the cleavable oligopeptide (and/or to the linker moiety L2, if any). The expression "release of the therapeutic agent (or marker) linked to a portion of the cleavable oligopeptide" is explained by the following example. If the cleavable oligopeptide moiety has Ala-Leu-Ala-Leu, the therapeutic agent is doxorubicin (whereby the therapeutic agent directly linked to the cleavable oligopeptide is Ala-Leu-Ala-Leu-doxorubicin) and the enzyme is CD10 (neprilysin), then this enzyme cleaves the sequence of amino acids between Ala-Leu-Ala and Leu, thus releasing a Leu-doxorubicin product (*i.e.,* the therapeutic agent linked to a part of the cleavable oligopeptide, the last Leu in the example).

The compounds of the invention comprise a therapeutic agent or marker moiety. "Therapeutic agent" is intended to mean a compound that, when present in a therapeutically effective amount, produces a desired therapeutic effect on a mammal, and whose action site is located or whose effect will be exerted on the surface of, or inside, target cells. By way of example, a therapeutic agent can be selected from the following group: a chemical agent, a polypeptide, a protein, an antibody, a nucleic acid, an antibiotic or a virus.

Said therapeutic agent is preferably an agent with anti-tumor, anti-angiogenic or anti-inflammatory therapeutic activity. The agent can have an extracellular target (for example a receptor) or an extracellular or intracellular action site. For example, a therapeutic agent according to the invention may be selected from vinca alkaloids such as vincristine, vinblastine, vindesine, vinorelbine; taxanes or taxoids such as paclitaxel, docetaxel, 10-deacetyltaxol, 7-*epi*-taxol, baccatin III, 7-xylosyltaxol, isotaxel; alkylating agents such as ifosfamide, melphalan, chloroaminophene, procarbazine, chlorambucil, thiophosphoramide, busulfan, dacarbazine (DTIC), mitomycins including mitomycin C, nitroso-ureas and derivatives thereof (for example, estramustine, BCNU, CCNU, fotemustine, streptonigrin); platinum derivatives such as cisplatin and the like (for example, carboplatin, oxaliplatin); antimetabolites such as methotrexate, aminopterin, 5-fluorouracil, 6-mercaptopurine, raltitrexed, cytosine arabinoside (or cytarabine), adenosine arabinoside, gemcitabine, cladribine, pentostatin, fludarabine phosphate, hydroxyureas; inhibitors of topoisomerase I or II such as the camptothecin derivatives (for example, irinotecan and topotecan or 9-dimethylaminomethyl-hydroxy-camptothecin hydrochloride), epipodophyllotoxins (for example etoposide, teniposide), amsacrine; mitoxantrone; L-canavanine; antibiotic agents such as anthracyclines and, for example, doxorubicin, THP-adriamycin, daunorubicin, idarubicin, rubidazone, pirarubicin, zorubicin and aclarubicin, the analogs of anthracyclines and, for example, epiadriamycin (4'epi-adriamycin or epirubicin) and mitoxantrone, bleomycins, actinomycins including actinomycin D, streptozotocin, calicheamycin, duocarmycins, combretastatin; L-asparaginase; hormones; pure inhibitors of aromatase; androgens, analog-antagonists of LH-RH; cytokines such as interferon alpha (IFN-alpha), interferon gamma (IFN-gamma), interleukin 1 (IL-1), IL-2, IL-4, IL-6, IL-10, IL-12, IL-15, tumor necrosis factor-alpha (TNF-alpha), IGF-1 antagonists (insulin-like growth factor); proteasome inhibitors; farnesyl-transferase inhibitors (FTI); epothilones; maytansinoids; discodermolide; fostriecin; inhibitors of tyrosine kinases such as STI 571 (imatinib mesylate); receptor tyrosine kinase inhibitors such as lapatinib, erlotinib, sorafenib, vandetanib, canertinib, PKI166, gefitinib, sunitinib, EKB-569; Bcr-Ab1 kinase inhibitors such as dasatinib, nilotinib, imatinib; aurora kinase inhibitors such as VX-680, CYC116, PHA-739358, SU-6668, JNJ-7706621, MLN8054, AZD-1152, PHA-680632; CDK inhibitors such as flavopirodol, seliciclib, E7070, BMS-387032; MEK inhibitors such as PD184352, U-0126; mTOR inhibitors such as CCI-779 or AP23573; Kinesin spindle inhibitors such as; SB 743921 or MK-0731; RAF/MEK inhibitors such as; Sorafenib, CHIR-265, PLX-4032, CI-1040, PD0325901 or ARRY-142886; bryostatin; L-779450; LY333531; velcade; ispinib; endostatins; proteins; peptides; antibodies; anti-inflammatory cytokines.

Particularly preferred therapeutic agents are doxorubicin, mitomycin-C and paclitaxel.

"Marker" is intended to mean a compound useful in the characterization of tumors or other medical conditions, for example, diagnosis, progression of a tumor, and assay of the factors secreted by tumor cells. A Marker is further defined as enzymes, antibodies, fluorescent or phosphorescent chemical molecules, and molecules that can be used in scintigraphy. Examples of markers include, but are not limited to, coumarin, 7-amido-trifluoromethyl coumarin, paranitroanilide, 8-naphthylamide and 4-methoxy naphthylamide, fluoresceine, biotin, rhodamine, tetramethylrhodamine, GFP (green fluorescent protein), agents that are used in scintigraphy as radioactive isotopes, and the derivatives of these compounds.

When the cleavable oligopeptide moiety of a compound of the invention is directly linked to the therapeutic agent or marker, i.e. when L2 in formula (II) is a covalent bond, said covalent bond between said oligopeptide moiety and said therapeutic agent or marker can then be produced at the N-terminal or C-terminal end of the cleavable oligopeptide, according to its orientation, or at any other site of the oligopeptide (for example at the lateral chain of one of the amino acids).

A direct chemical reaction between a therapeutic agent and an oligopeptide is possible when each possesses a functional group capable of reacting with the other. For example, a nucleophilic group, such as an amino or sulfhydryl group, can be reacted with a carbonyl-containing group, such as an anhydride or an acid halide, or with an alkyl group containing a good leaving group (e.g., a halide). Examples of bonds between an oligopeptide and a therapeutic agent may be found in PCT Publication No WO 96/05863, which is incorporated herein by reference. Preferred bonds are amide, ester or thioester, carbonate, carbamate, hydrazone, oxime, ether and thioether, thiazolidine or oxazolidine, imidazole or disulfide.

The compounds of the invention present a TA derived moiety directly or indirectly linked to an oligopeptide moiety, said oligopeptide moiety being directly or indirectly linked to a therapeutic agent or marker. A direct link between two parts of the molecule means that one of these parts comprises a reactive group which can allow formation of a covalent bond with a reactive group on the other part.

The terms "indirect linkage" or "indirectly linked" mean that two portions of the compound of the invention are covalently linked through a linking moiety. If the TA derived moiety and the cleavable oligopeptide are indirectly linked, then a linking moiety L₁ is present. In the same way, if the cleavable oligopeptide moiety and the therapeutic agent or marker are indirectly linked, then a linking moiety L₂ is present. According to one embodiment, the compounds of the invention have the TA derived moiety and the cleavable oligopeptide moiety indirectly linked. In another embodiment, the compounds of the invention have indirect linkage of the cleavable oligopeptide to the therapeutic agent or marker. In yet another embodiment, the compounds of the invention have indirect linkage of the TA derived moiety and the cleavable oligopeptide and indirect linkage of the cleavable oligopeptide to the therapeutic agent or marker.

In a particular embodiment of the invention, the orientation of the cleavable oligopeptide may be reversed so that the TA derived moiety is indirectly linked to the cleavable oligopeptide at the C-terminus of the cleavable oligopeptide and the therapeutic agent or marker is directly or indirectly linked to the N-terminus of the cleavable oligopeptide. In a particular embodiment of the invention, a first attachment site of the cleavable oligopeptide may be the C-terminus of the cleavable oligopeptide and a second attachment site of the cleavable oligopeptide may be the N-terminus of the cleavable oligopeptide.

The term "linking moiety" includes di- and multivalent organic radicals independently selected from substituted or unsubstituted alkyl, substituted or unsubstituted heteroalkyl and substituted or unsubstituted aryl, aldehydes, acids, esters and anhydrides, ketone, acyl halide, quinone, alkylene, cycloalkyl, heterocycloalkyl, sulphydryl or carboxyl groups, such as maleimido acid derivatives, and succinimido derivatives or may be derived from succinimidyl esters and the like. Particularly, the linking moiety can also include aminobenzyl spacer, elongated electronic cascade and cyclisation spacer systems, cyclisation elimination spacers, such as ω-amino aminocarbonyls, and p-aminobenzyoxycarbonyl linkers (de Groot et al., J. Med. Chem. 43, 3093 (2000)).

The linking moiety may comprise a short amino acid sequence from 1 to 10 amino acid residues that is not cleaved by the enzyme cleaving the cleavable oligopeptide moiety of a compound of formula (II). Accordingly the linking moiety comprises identical or different amino acids selected from natural amino acids in conformation L or D, non genetically encoded amino acids or amino acids that cannot be recognized by enzymes (e.g. peptidases) that are present in the circulatory structure, such as beta- or gamma-amino acids or the like. The term "natural amino acids in conformation D" denotes the amino acids that are normally coded by the genetic code but that instead of being naturally in conformation L are in conformation D. Generally, the non genetically encoded amino acids can be prepared by synthesis or can be derived from a natural source. Preferred among the natural amino acids in conformation D are the hydrophilic amino acids that are selected from among: D-serine, D-thréonine, D-glutamine, D-asparagine, D-aspartic acid, D-glutamic acid, D-lysine, D-arginine and D-histidine. Particularly preferred amino acids in conformation D are D-serine and D-threonine. By way of example, the linking moiety may comprise a stretch of identical amino acids that are selected from among: (L-serine)x, (D-serine)x, or (L-threonine)x and (D-threonine)x, where x is an integer between 1 and 10, preferably x = 2 or 4.

Linking moieties have also been disclosed in de Groot et al., J. Med. Chem. 42, 5277 (1999); de Groot et al., J. Org. Chem. 43, 3093 (2000); de Groot et al., J. Med. Chem. 66, 8815, (2001); PCT Publication No WO 02/083180; Carl et al. , J. Med. Chem. Lett. 24, 479 (1981); Dubowchik et al., Bioorg & Med. Chem. Lett. 8, 3347 (1998).

It will be evident to those skilled in the art that a variety of bifunctional or polyfunctional reagents, both homo- and hetero-functional can be employed as a linking moiety, such as those described in the catalog of the Pierce Chemical Co., Rockford, III., 2006.

Preferably, the linking moiety according to this invention consists of, or alternatively comprises, at least one moiety selected from: sequences of amino acids; peptidomimetic agents; pseudopeptides; peptoids; substituted alkyl, aryl alkylene or arylalkyl chains; polyalkyl glycols; polysaccharides; polyamino; polyols; polycarboxylates; and poly(hydroxy)esters.

The linking may space the 1,2,3,4-cyclobutanetetracarboxylic acid derived moiety or the therapeutic agent or marker from the oligopeptide in order to avoid interference in the interaction of the oligopeptide with enzymes associated with target cells. A linking moiety can also serve to allow or increase the coupling efficiency between two portions of the compounds of the invention. For example, if the therapeutic agent or marker (D in formula II) does not comprise a group allowing its direct binding to the cleavable peptide moiety (X in formula II), a linker moiety L2 comprising reactive groups compatible with both X and D moieties may be inserted for coupling these two parts.

In a preferred embodiment, the therapeutic agent or marker is directly linked to the cleavable oligopeptide moiety. It will be evident for one skilled in the art that this embodiment requires that the therapeutic agent or marker and the cleavable oligopeptide moiety present radicals which can allow formation of a covalent bond.

The term "alkyl" by itself or as part of another substituent, means, unless otherwise stated, a straight or branched chain, or cyclic hydrocarbon radical, optionally interrupted by at least one heteroatom including O, N, Si and S (as defined below), or combination thereof, which may be fully saturated, mono-or polyunsaturated and can include di- and multivalent radicals, having the number of carbon atoms designated (i.e. C₁-C₁₀ means one to ten carbons). Examples of saturated hydrocarbon radicals of formula CₙH₂ₙ₊₁ include, but are not limited to, groups such as methyl, ethyl, n-propyl, isopropyl, n-butyl, t-butyl, isobutyl, sec-butyl, cyclohexyl, (cyclohexyl) methyl, cyclopropylmethyl, homologs and isomers of, for example, n-pentyl, n-hexyl, n-heptyl, n-octyl, and the like. An unsaturated alkyl group is one having one or more double bonds (e.g., alkenyl groups of formula CₙH₂ₙ) or triple bonds (e.g., alkynyl groups of formula CₙH₂ₙ₋₂). Examples of unsaturated alkyl groups include, but are not limited to, vinyl, 2-propenyl, crotyl, 2-isopentenyl, 2-butadienyl, 2,4-pentadienyl, 3(1,4-pentadienyl), ethynyl, 1-and 3-propynyl, 3-butynyl, and the higher homologs and isomers. The term "alkyl" unless otherwise noted, is also meant to include those derivatives of alkyl defined in more detail below, such as "heteroalkyl". Alkyl groups, which are limited to hydrocarbon groups are termed "homoalkyl".

The term "alkyl" by itself or as part of another substituent includes mono- or divalent radical derived from an alkane. Among the divalent radical, one can cite as non limiting examples -CH₂-, -CH₂CH₂-, -CH₂CH₂CH₂-, -CH₂CH₂CH₂CH₂-.

Typically, an alkyl group will have from 1 to 24 carbon atoms, with those groups having 10 or fewer carbon atoms being preferred in the present invention. A "lower alkyl" is a shorter chain alkyl group, generally having eight or fewer carbon atoms.

The term "alkenyl" by itself or as part of another substituent means a divalent radical derived from an alkane, as exemplified, but not limited, by -CH₂=CH₂-, and further includes those groups described below as "heteroalkylene". Typically, an alkylene group will have from 2 to 24 carbon atoms, with those groups having 10 or fewer carbon atoms being preferred in the present invention. A "lower alkylene" is a shorter alkylene group, generally having eight or fewer carbon atoms.

The term "heteroalkyl" by itself or in combination with another term, means, unless otherwise stated, a stable straight or branched chain, or cyclic hydrocarbon radical, or combinations thereof, consisting of the stated number of carbon atoms and at least one heteroatom selected from the group consisting of O, N, Si and S, and wherein the nitrogen, carbon and sulfur atoms may optionally be oxidized and the nitrogen heteroatom may optionally be quaternized. The heteroatom(s) O, N and S and Si may be placed at any interior position of the heteroalkyl group or at the position at which the alkyl group is attached to the remainder of the molecule. Examples include, but are not limited to, -CH₂-O-CH₃, ethyleneglycol and the like. Up to two heteroatoms may be consecutive, such as, for example, -CH₂-NH-OCH₃ and -CH₂-O-S(CH₃)₃.

The term "heteroalkyl" may encompass poly(ethylene glycol) and its derivatives. Still further, for divalent alkyl and heteroalkyl groups, no orientation of the linking group is implied by the direction in which the formula of the group is written. For example, the formula -C(O)₂R'- represents both -C(O)₂R'- and -R'C(O)₂-.

The term "lower" in combination with the terms "heteroalkyl" refers to a moiety having from 1 to 8 carbon atoms.

The terms "alkoxy", "alkylamino" and "alkylthio" (or "thioalkoxy") are used in their conventional sense, and refer to those alkyl groups attached to the remainder of the molecule via an oxygen atom, an amino group, or a sulfur atom, respectively.

In general, an "acyl substituent" is also selected from the group set forth above. As used herein, the term "acyl substituent" refers to groups attached to, and fulfilling the valence of a carbonyl carbon that is either directly or indirectly attached to the compounds of the present invention.

The terms "cycloalkyl" and "heterocycloalkyl", by themselves or in combination with other terms, represent, unless otherwise stated, cyclic versions of substituted or unsubstituted "alkyl" and substituted or unsubstituted "heteroalkyl", respectively. Additionally, for heterocycloalkyl, a heteroatom can occupy the position at which the heterocycle is attached to the remainder of the molecule. Examples of cycloalkyl include, but are not limited to, cyclopentyl, cyclohexyl, 1-cyclohexenyl, 3-cyclohexenyl, cycloheptyl, and the like. Examples of heterocycloalkyl include but are not limited to, 1-piperidinyl, 3-piperidinyl, tetrahydrofuran-2-yl and the like. The heteroatoms and carbon atoms of the cyclic structures are optionally oxidized.

The terms "halo" or "halogen" by themselves or as part of another substituent, mean, unless otherwise stated, a fluorine, chlorine, bromine, or iodine atom. Additionally, terms such as "haloalkyl" are meant to include monohaloalkyl and polyhaloalkyl. For example, the term "halo(C₁-C₄)alkyl" is meant to include, but is not limited to, trifluoromethyl, 2,2,2-trifluoroethyl, 4-chlorobutyl, 3-bromopropyl, and the like.

The term "aryl" means, unless otherwise stated, a substituted or unsubstituted polyunsaturated, aromatic, hydrocarbon substituent which can comprise a single ring or multiple rings (preferably from 1 to 3 rings) which are fused together or linked covalently. The term "heteroaryl" refers to aryl groups (or rings) that contain from one to four heteroatoms selected from N, O, and S, wherein the nitrogen, carbon and sulfur atoms are optionally oxidized, and the nitrogen atom(s) is optionally quaternized. A heteroaryl group can be attached to the remainder of the molecule through a heteroatom. Non-limiting examples of aryl and heteroaryl groups include phenyl, 1-naphthyl, 4-biphenyl, 1-pyrrolyl, 2-imidazolyl, pyrazinyl, 2-oxazolyl, 2-pyridyl, 4-pyrimidyl, 5-benzothiazolyl, and the like.

Substituents for each of the above noted aryl and heteroaryl ring systems are selected from the group of acceptable substituents described below. "Aryl" and "heteroaryl" also encompass ring systems in which one or more non-aromatic ring systems are fused, or otherwise bound, to an aryl or heteroaryl system. For brevity, the term "aryl" when used in combination with other terms (e.g., aryloxy, arylthioxy, arylalkyl) includes both aryl and heteroaryl rings as defined above.

Thus, the term "arylalkyl" is meant to include those radicals in which an aryl group is attached to an alkyl group (e.g., benzyl, phenethyl, pyridylmethyl and the like) including those alkyl groups in which a carbon atom (e.g., a methylene group) has been replaced by, for example, an oxygen atom (e.g., phenoxymethyl, 2-pyridyloxymethyl, 3-(1-naphthyloxy)propyl, and the like).

Each of the above radicals (e.g., "alkyl", "heteroalkyl", "aryl" and "heteroaryl") include both substituted and unsubstituted forms of the indicated radical. Preferred substituents for each type of radical are provided below. Substituents are independently the same or different and are preferably alkyl group, alkylene group, heteroalkyl group, alkoxy group, alkylamino group, alkylthio group, acyl group, cycloalkyl and heterocycloalkyl group, aryl, heteroaryl group, halogen.

The functional groups on the linking moiety used to form covalent bonds may correspond to different types of functional groups. For example, it will be evident to those skilled in the art that coupling can be effected through functional groups such as amino, carboxyl, carbonyl, hydrazine, hydroxylamino, quinone, hydroxyl, thiol, halide, imine, isocyanate, nitrile, phosphate, sulfonic, sulfhydryl, maleimido groups. Functional groups have also been disclosed in March's Advanced Organic Chemistry: Reactions, Mechanisms and Structure (6th edition by Michael B. Smith and Jerry March, edition Wiley).

By way of example, the TA derived moiety can be linked to a suitable cleavable oligopeptide moiety either directly or indirectly via a linking moiety L₁. If present, the linking moiety L₁ according to the present invention covalently links the TA derived moiety to the cleavable oligopeptide. It is preferably hydrophilic and stable in the circulatory structure (e.g. the blood circulation or bloodstream). A linking moiety is "stable in the circulatory structure" when less than about 20%, preferably less than about 10%, preferably even less than about 2% (by moles), of the linking moiety is degraded or cleaved (in particular by enzymes) in the circulating blood of a mammal or during its preservation in human blood at about 37°C, for more than about 2 hours. More preferably the linker is not degraded or cleaved (in particular by enzymes) in the circulating blood or during its preservation in human blood at about 37°C, for more than about 2 hours. In general, the cleavable oligopeptide moiety can be conjugated to the TA derived moiety, for example, by any suitable technique, with appropriate consideration of the need for pharmacokinetic stability and reduced overall toxicity to the patient.

By way of example, a therapeutic agent or marker can be linked (i.e. conjugated or coupled) to the cleavable oligopeptide moiety of the invention by any suitable technique known from one skilled in the art, with appropriate consideration of the need for pharmacokinetic stability and reduced overall toxicity to the patient. A therapeutic agent or marker can be linked to the oligopeptide either directly or indirectly via a linking moiety L₂.

The linking moiety L₂ may have several functions such as to facilitate the cleavage between the oligopeptide and the therapeutic agent or marker, to provide a suitable chemical bonding means between the oligopeptide and the therapeutic agent or marker, to improve the synthesis process of the compound of the invention, to improve the physical properties of the substance of the therapeutic agent or marker, or to provide an additional mechanism for the intracellular or extracellular release of the therapeutic agent or marker at or near the target cells. Advantageously it can be desirable to use a linking moiety L₂ which is cleavable during or upon penetration into a cell of the therapeutic agent linked to a portion of the cleavable oligopeptide, or which is gradually cleavable over time in the extracellular environment. The mechanisms for the intracellular release of a therapeutic agent from these linking moieties comprise cleavage by reduction of a disulfide bond, by irradiation of a photolabile bond, by hydrolysis of derivatized amino acid side chains and acid-catalyzed hydrolysis.

More preferred linking moieties L₂ are *N,N'*-ethylenediamine, ethanolamine, aminocaproic acid, 4-aminobenzyl alcohol, 6-Amino-1-hexanol, 1,3-diaminopropane, cysteamine, 1,4-diaminobutane, *N*-methyl-1,3-propanediamine, *N-*methylethylenediamine, 4-aminobenzylamine.

The invention encompasses also pharmaceutically acceptable basic or acidic addition salts, hydrates, solvates, or stereoisomers of said compounds of the invention.

The terms "pharmaceutically acceptable salts" includes salts of the compounds of the invention which are prepared with non toxic acids or bases, depending on the particular substituents found on the compounds described herein. When compounds of the present invention contain relatively acidic functionalities, base addition salts can be obtained by contacting the neutral form of such compounds with a sufficient amount of the desired base, either neat or in a suitable inert solvent. Examples of pharmaceutically acceptable base addition salts include sodium, potassium, calcium, ammonium, organic amino, or magnesium salt, or a similar salt. When compounds of the present invention contain relatively basic functionalities, acid addition salts can be obtained by contacting the neutral form of such compounds with a sufficient amount of the desired acid, either neat or in a suitable inert solvent. Examples of pharmaceutically acceptable acid addition salts include those derived from inorganic acids like hydrochloric, hydrobromic, nitric, carbonic, monohydrogencarbonic, phosphoric, monohydrogenphosphoric, dihydrogenphosphoric, sulfuric, monohydrogensulfuric, hydriodic, or phosphorous acids and the like, as well as the salts derived from relatively non toxic organic acids like acetic, propionic, isobutyric, maleic, malonic, benzoic, succinic, suberic, fumaric, lactic, mandelic, phthalic, benzenesulfonic, p-tolylsulfonic, citric, tartaric, methanesulfonic, and the like. Also included are salts of amino acids such as arginate and the like, and salts of organic acids like glucuronic or galactunoric acids and the like. Certain specific compounds of the present invention contain both basic and acidic functionalities that allow the compounds to be converted into either base or acid addition salts.

The present invention also relates to methods for preparing compounds of formula (II) as defined above.

The compounds of the invention may be prepared by any method known in the art, and in particular as described in the examples of the present application.

For example, the cleavable oligopeptide moiety can be prepared using conventional solution- or solid-phase peptide synthesis methods. This peptide can then be reacted directly with the therapeutic agent or marker moiety and then a TA derived moiety can be reacted with the resulting product in order to form compounds of the invention.

The skilled person is capable of preparing a wide variety of compounds of formula (II) utilising a variety of linker moieties. As stated above, an appropriate group on the therapeutic agent or marker may be selected for attachment to the cleavable oligopeptide moiety and if desired a linker L2 joined to said therapeutic agent or marker or to said oligopeptide, or both, prior to their coupling. Alternatively, the therapeutic agent or marker may also be modified so as to allow conjugation.

Advantageously, the combination of the 1,2,3,4-cyclobutanetetracarboxylic acid derived moiety with a cleavable oligopeptide (i.e. with an extracellular activation process by enzymes associated with target cells) allows the therapeutic use of the compounds of the invention to improve the delivery of a therapeutic agent selectively within the target cell environment (e.g., target tissue).

The compounds of the invention are administered to the patient, carried through the blood stream in a stable form, and are modified by at least one enzyme specific of the target cell when in the vicinity of said target cell. Since the enzyme activity is only minimally, or preferably not, present within the extracellular vicinity of normal cells, the cleavable oligopeptide moiety is generally poorly, or not, cleaved outside target tissues and the therapeutic agent or marker reach normal cells only minimally.

The compounds of the invention are preferably stable in the circulatory structure. "Stable in the circulatory structure" means that less than about 20%, preferably less than about 2% (by mole), of a compound of the invention is degraded or cleaved in the circulating blood of a mammal or during its preservation in human blood at about 37°C for more than about 2 hours. The term "circulatory structure" is defined as body fluids, more particularly blood. In a specific embodiment the "circulatory structure" comprises tissues of the circulatory system.

The compounds of the invention are non toxic for healthy cells, non-coagulating (*i*.*e*. overcomes pro-coagulating properties of the therapeutic agent linked to the cleavable oligopeptide) and hindering (*i.e.*, preventing the therapeutic agent from acting on a cell until it has been released from the compound).

Another aspect of the invention provides a composition that comprises, or alternatively consists of, as an active ingredient, at least one compound of the invention.

In still another aspect, the invention contemplates the use of such compositions for the formulation and the preparation of biological, pharmaceutical, cosmetic, agricultural, diagnostic or tracing products.

In one aspect, the invention provides a pharmaceutical composition that comprises, or alternatively consists of, at least one compound according to the present invention that can be combined with a pharmaceutically acceptable carrier (vehicle, vector, diluent or excipient). The terms "pharmaceutically acceptable", as they refer to carrier (vehicle, vector, diluent or excipient), mean that the materials may be administered to a subject without subsequent production of undesirable physiological effects to a degree that would prohibit administration of the composition.

The compounds of the invention are useful to treat a mammalian subject, preferably a human subject. A subject can be treated with a pharmaceutically effective amount of a compound according to the invention. The expression "pharmaceutically effective amount" or "effective amount of means an amount that can induce the biological or medical response of a tissue, system, animal or human as expected by the research worker or the physician in attendance.

The compositions defined above can also comprise, or be combined with, at least one other medicinal active ingredient or at least one adjuvant that is well known to one skilled in the art, such as for example vitamins or anti-oxidizing agents, to be used in conjunction with a compound according to the invention to improve and/or to extend the treatment. Indeed, the compositions of the invention are particularly useful in that they have a very low toxicity.

The pharmaceutical compositions according to the invention can be used *in vivo* for preventive or curative purposes of specific diseases or disorders. Non-limiting examples of diseases or disorders for which the pharmaceutical formulations according to the invention may be used include viral infections, cancers, metastases, cellular apoptosis disorders, degenerative diseases, tissue ischemia, infectious diseases of a viral, bacterial or fungal nature, inflammation disorders and pathological neo-angiogenesis, metabolic disorders.

The term "cancer" refers to any of a number of diseases that are characterized by uncontrolled, abnormal proliferation of cells, the ability of affected cells to spread locally or through the bloodstream and lymphatic system to other parts of the body *(i.e.,* metastases), as well as any of a number of characteristic structural and/or molecular features. A "cancerous cell" or "cancer cell" is understood as a cell having specific structural properties, that can lack differentiation and be capable of invasion and metastasis. Examples of cancers are lung (*i*.*e*. small cell, non-small cell, bronchic cancers), pancreas, ovarian, breast, prostate, liver, kidney, colon, head, brain, bone, stomach, bladder and non-Hodgkin cancers (see DeVita, V. et al. (eds.), 2005, Cancer Principles and Practice of Oncology, 6th. Ed., Lippincott Williams & Wilkins, Philadelphia, PA; this reference is herein incorporated by reference in its entirety for all purposes).

A further aspect of the invention provides a method for the treatment of cellular proliferative and/or differentiative disorders, disorders associated with bone metabolism, immune disorders, hematopoietic disorders, cardiovascular disorders, liver disorders, kidney disorders, muscular disorders, neurological disorders, hematological disorders, inflammatory diseases, viral diseases, pain or metabolic disorders, preferably for treating cancers comprising administering, preferably parenterally and more preferably intravenously, to a patient in need of such treatment an effective amount of a composition of the invention.

"Treating" or "treatment" includes the administration of the compositions or compounds of the present invention to a patient who has a disease or disorder (e.g., cancer or metastatic cancer), a symptom of disease or disorder or a predisposition toward a disease or disorder, with the purpose to cure, heal, alleviate, relieve, alter, remedy, ameliorate, improve or affect the disease or disorder, the symptoms of the disease or disorder, or the predisposition toward disease. "Treating" or "treatment" of cancer or metastatic cancer using the methods of the present invention refers to the treatment or amelioration or prevention of a cancer, including any objective or subjective parameter such as abatement, remission, diminishing of symptoms or making the disease condition more tolerable to the patient, slowing in the rate of degeneration or decline, or making the final point of degeneration less debilitating. The treatment or amelioration of symptoms can be based on objective or subjective parameters, including the results of an examination by a physician. Accordingly, the term "treating" includes the administration of the compounds or agents of the present invention to prevent or delay, to alleviate, or to arrest or inhibit development of the symptoms or conditions associated with neoplastic disease. The term "therapeutic effect" refers to the reduction, elimination, or prevention of the disease, symptoms of the disease, or side effects of the disease in the subject.

The compounds of the invention are generally useful for the treatment of many medical conditions including cancer, neoplastic diseases, tumors, inflammatory diseases, and infectious diseases. Examples of preferred diseases for treatment are breast cancer, colorectal cancer, liver cancer, lung cancer such as small cell or non-small cell, bronchic cancers, prostate cancer, ovarian cancer, brain cancer, and pancreatic cancer. colon cancer, head, stomach and neck cancers, bladder cancer, non-Hodgkin's lymphoma cancer, melanoma, leukaemia, neuroblastoma, or glioblastoma.

More specifically, since several specific tumor types have been identified as being positive for CD10, treatment for these types of tumors is especially advantageous with the compounds disclosed herein. Specifically, treatment for one of the following tumor types may be effected: B-cell lymphoblastic leukemia, T-cell lymphoblastic leukemia, lymphoma, including Hodgkin's lymphoma and non-Hodgkin's lymphoma, follicular lymphoma, Burkitt lymphoma, melanoma, ocular melanoma, cutaneous melanoma, colon adenocarcinomas, hepatocellular carcinomas, renal cell carcinoma, ovarian carcinoma, prostate adenocarcinoma, liver carcinoma, transitional cell carcinoma, pancreatic adenocarcinoma, lung carcinoma, breast carcinoma and colon carcinoma.

In yet another aspect, the invention provides the use of at least one compound of the invention for the manufacture of a medicament for treating or preventing a number of disorders, including cellular proliferative and/or differentiative disorders, disorders associated with bone metabolism, immune disorders, hematopoietic disorders, cardiovascular disorders, liver disorders, kidney disorders, muscular disorders, neurological disorders, hematological disorders, inflammatory diseases, viral diseases, pain or metabolic disorders, preferably for treating cancers.

Furthermore, a particular embodiment of the invention relates to the use of at least one compound of formula II as defined above for the manufacture of a medicament for the treatment of cancers or infectious diseases.

Another particular embodiment of the invention relates to the use of a compound of formula II as defined above for the preparation of a medicament for treating breast cancers, colorectal cancers, liver cancers, lung cancers such as small cell or non-small cell, bronchic cancers, prostate cancers, ovarian cancers, brain cancers, and pancreatic cancers, colon cancers, head and neck cancers, stomach cancers, bladder cancers, non-Hodgkin's lymphomas, melanomas, leukaemias, neuroblastomas, or glioblastomas.

The administration of the compounds according to the invention can be done by any of the administration methods accepted for the therapeutic agents and generally known in the art. These processes include, but are not limited to, systemic administration, for example orally, nasally, parenterally or topical administration, for example by transdermal means or else by central administration, for example by an intracranial surgical path, or else by intraocular administration.

The parenteral administration is done generally by subcutaneous, intramuscular or intravenous injection, or by perfusion. The injectable compositions can be prepared in standard forms, either in suspension or liquid solution or in solid form that is suitable for an extemporaneous dissolution in a liquid.

A possibility for parenteral administration uses the installation of a system with slow release or extended release that ensures the maintenance of a constant dose level.

Based on the administration method provided, the compounds of the invention can be in solid, semi-solid or liquid form.

For solid compositions, such as tablets, pills, powders or granules in the free state or included in capsules, the active ingredient can be combined with excipients, such as: a) diluents, for example lactose, dextrose, sucrose, mannitol, sorbitol, cellulose and/or glycine; b) lubricants, for example silica, talc, stearic acid, its magnesium or calcium salt and/or polyethylene glycol; c) binders, for example magnesium silicate and aluminum silicate, starch paste, gelatin, tragacanth gum, methyl cellulose, carboxymethyl cellulose that contains soda and/or polyvinyl pyrrolidone; if necessary, d) disintegrating agents, for example starch, agar, alginic acid or its sodium salt, or effervescent mixtures; and/or e) absorbents, coloring agents, aromatizing agents and sweetening agents.

For semi-solid compositions, such as suppositories, the excipient can be, for example, a fatty emulsion or suspension or can be based on polyalkylene glycol, such as polypropylene glycol.

The liquid compositions, in particular those intended for injection or to be included in a soft capsule, can be prepared by, for example, dissolution, dispersion, etc., of the compound according to the invention in a pharmaceutically pure solvent such as, for example, water, a saline solution of sodium chloride (NaCl), the physiological serum, aqueous dextrose, glycerol, ethanol, an oil and the like.

The compounds according to the invention can also be administered in the form of systems for release of the liposome or lipoplex type, such as in the form of small unilamellar vesicles, large unilamellar vesicles and multilamellar vesicles. The liposomes can be formed from a variety of phospholipids, containing in particular cholesterol, stearylamine or phosphatidylcholines.

The compositions according to the invention should be sterilized and/or can contain one or more of non-toxic adjuvants and auxiliary substances such as agents for preservation, stabilization, wetting or emulsification; agents that promote dissolution; and salts to regulate osmotic pressure and/or buffers. In addition, they can also contain other substances that offer a therapeutic advantage. The compositions are prepared, respectively, by standard processes of mixing, granulation or coating well known to those skilled in the art.

The dosage for the administration of compounds according to the invention is selected according to a variety of factors including the type, strain, age, weight, sex and medical condition of the subject; the severity of the condition to be treated; the method of administration; the condition of the renal and hepatic functions of the subject and the nature of the particular compound or salt that is used. A normally experienced physician or veterinarian will easily determine and prescribe the effective amount of the desired compound to prevent, disrupt or stop the progress of the medical condition that is to be treated.

A pharmaceutical composition as set forth above can contain from about 0.1 to about 100 % (by weight) of at least one compound of the invention.

By way of examples, when administered parenterally, the effective levels of the compounds according to the invention will be in the range of from about 0.002 mg to about 500 mg per kg of body weight and per day.

The compounds according to the invention can be administered in the form of single daily doses, or the total daily dosage can be administered in two, three, four or more doses per day.

In a further aspect, the invention provides a diagnostic agent comprising or alternatively consisting of at least one compound of formula II according to the present invention wherein D is a marker. Such a diagnostic agent can be used either *in vitro* or *in vivo.*

In yet another aspect, the invention provides a diagnostic kit that comprises the above diagnostic agent. More particularly, the diagnostic kit comprises, in one or more containers, a predetermined amount of a composition according to the invention.

Further advantages and characteristics of the invention will be apparent for one skilled in the art from the following examples, given by way of illustration and which are not intended to be limiting, and in which reference will be made to the accompanying drawings.

It will be clear that the invention may be practiced otherwise than as particularly described in the foregoing examples. Numerous modifications and variations of the present invention are possible in light of the above teachings and, therefore, are within the scope of the appended claims.

### EXAMPLES

### Materials

### 1,2,3,4-cyclobutanetetracarboxylic acid was purchased from Sigma-Aldrich (France)

### Peptides:

- Fmoc-Ala-Leu-Ala-Leu-OH (Fmoc-ALAL-OH): synthesis as described below
- MeO-Suc-βALAL-OH was supplied by PeptySyntha (Brussels, Belgium)
- tBu-Suc- βALAL-OH was supplied by NeoMPS (Strasbourg, France)

### Drugs:

- mitomycin C (MMC): purchased from Kangbaoxiang Laboratories (Zhengzhou, China)
- paclitaxel: purchased from Hauser (Denver, CO, USA)
- doxorubicin (dox): purchased from Meiji Seika Kaisha Ltd. (Tokyo, Japan)

### Cell lines:

- HCT 116 colon cancer cell line; ATCC Number: CCL-247
- LS 174T human colorectal carcinoma cell line; ATCC Number: CC1-188

### I.1 Method of synthesis of TetraAcid-ALAL-doxorubicin

### I.1.1 Synthesis ofFmoc-ALAL-OH

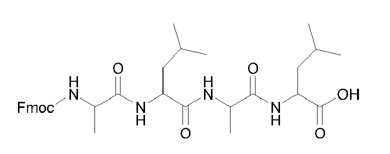

The Wang resin-Leu-Fmoc (5 g, 3.65 mmol) was swelled in dichloromethane (15 mL) for 5 minutes then in dimethylformamide (15 mL) for 5 minutes. The Fmoc group was removed by treatment with piperidine (4 mL) in dimethylformamide (16 mL) for 1 minute, the resin was then filtered, followed by the same treatment for 30 minutes. The resin was washed alternately with dimethylformamide (15 mL) and dichloromethane (15 mL) three times. Fmoc-Alanine-OH (3.408 g, 10.95 mmol) and 2-(6-chloro-1H-benzotriazole-1-yl)-1,1,3,3-tetramethyluronium hexafluorophosphate (4.074 g, 9.85 mmol) were solubilised in dimethylformamide (15 mL) and added to the resin followed by *N*,*N'*-diisopropylethylamine (3.8 mL, 21.9 mmol). The resin was then shaken for 30 minutes and washed alternately with dimethylformamide (15 mL) and dichloromethane (15 mL) three times. The solvent was removed and a second coupling was performed with Fmoc-Alanine-OH (2.272 g, 7.3 mmol), 2-(6-chloro-1H-benzotriazole-1-yl)-1,1,3,3-tetramethyluronium hexafluorophosphate (2.718 g, 6.57 mmol) and *N,N'-*diisopropylethylamine (2.5 mL, 14.6 mmol). The resin was then shaken for 30 minutes and washed alternately with dimethylformamide (15 mL) and dichloromethane (15 mL) three times. The same protocol was repeated with Fmoc-Leucine-OH then with Fmoc-Alanine-OH. After the last coupling, the resin was washed with dimethylformamide (15 mL), then dichloromethane (15 mL) three times and dried. The peptide was cleaved from the resin with a solution of trifluoroacetic acid / triisopropylsilane / dichloromethane (95/1/4 v/v) (15 mL) two times during 30 minutes. The solvent was evaporated and the residue precipitated in *tert*-butyl methyl ether (100 mL). Dichloromethane was then added to the precipitate and the resulting mixture was co-evaporated several times to obtain a white solid which was used without further purification.
Purity: 90 % (determined by HPLC at 214 nm).
MS (ES⁺): 610 [MH]⁺

### I.1.2 Synthesis of ALAL-doxorubicin

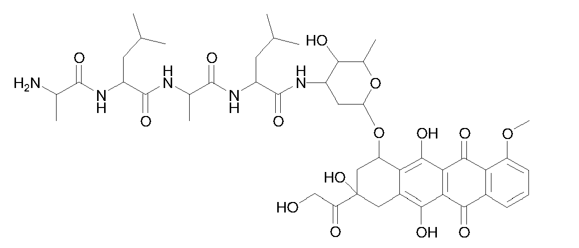

Doxorubicin (0.479 g, 0.825 mmol, 1 eq) was solubilised in dimethylformamide (7 mL). A solution of Fmoc-ALAL-OH (0.600 g, 1 mmol, 1.2 eq) in dimethylformamide (1.4 mL) and H₂O (1.6 mL) was added to the doxorubicin solution followed by *N,N'-*diisopropylethylamine (0.461 mL, 2.64 mmol, 3.2 eq). The solution was stirred at 4°C for 10 minutes. A solution of *O*-(7-Azabenzotriazol-1-yl)-*N*,*N*,*N',N'*-tetramethyluronium hexafluorophosphate (0.376 g, 1 mmol, 1.2 eq) in dimethylformamide (1.6 mL) was added dropwise at 4°C. The solution was stirred for 40 minutes at room temperature and directly precipitated in *tert*-butyl methyl ether (100 mL) at 4°C. The resulting red oil was evaporated. To the red oil was added a solution of dimethylformamide (10 mL) and piperidine (4.2 mL, 41.25 mmol, 50 eq) and the solution was stirred for exactly 5 minutes at room temperature. The solution was directly quenched with HC1 6N (7 mL, 42 mmol, 50 eq) at 4°C (added slowly due to the exothermic nature of the reaction). The pH of the solution was adjusted to 6 with HC1 1N. The resulting red solution was precipitated in *tert*-butyl methyl ether (200 mL) at 4°C three times and the resulting red solid filtered and dried.

### I.1.3 Synthesis of TetraAcid-ALAL-doxorubicin

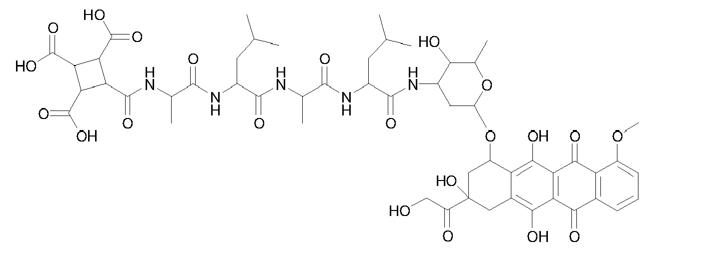

ALAL-doxorubicin (0.400 g, 0.44 mmol, 1 eq) was dissolved in dimethylformamide (10 mL). *N,N'*-diisopropylethylamine (0.230 mL, 1.32 mmol, 3 eq) was added followed by 1,2,3,4-cyclobutanetetracarboxylic acid (1.021 g, 4.4 mmol, 10 eq). Then 2-(6-chloro-1H-benzotriazole-1-yl)-1,1,3,3-tetramethyluronium hexafluorophosphate (0.237 g, 0.58 mmol, 1.3 eq) was added. The solution was stirred at room temperature for 30 minutes. Dichloromethane (30 mL) and water (15 mL) were added, the aqueous phase alkalinised to pH 8.5 with NaOH 1M and extracted four times with dichloromethane. The aqueous layer was purified by flash chromatography with silica C₁₈ (H₂O/acetonitrile 90/10 to 60/10 v/v). The fractions were analyzed by HPLC and the pure fractions were pooled and lyophilized to generate a red solid.
Purity: 96 % (determined by HPLC at 214 nm).
MS (ES⁺): 1127 [MH]⁺

### I.2 Method of synthesis of Suc-βALAL-doxorubicin

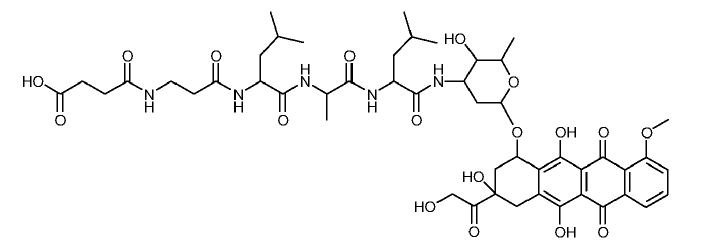

Suc-βALAL-doxorubicin was generated as previously described in Fernandez AM et al., J Med Chem 44:3750-3753 (2001).

### I.3 Method of synthesis of Suc-ALAL-doxorubicin

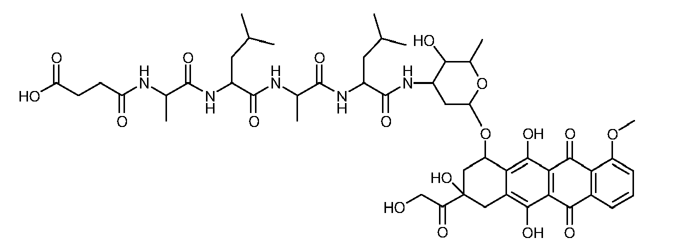

Suc-ALAL-doxorubicin was generated in a similar manner to that describe for Suc-βALAL-doxorubicin.

### I.4 Method of synthesis of TetraAcid-ALAL-mitomycin C

### I.4.1 Synthesis of Fmoc-ALAL-mitomycin C

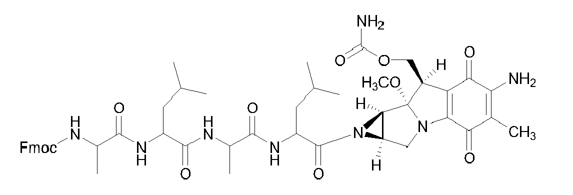

Mitomycin C (0.5 g, 1.5 mmol, 1 eq) was dissolved in dimethylformamide (20 mL) and water (5 mL) at +4°C. Fmoc-ALAL-OH (1.82 g, 3 mmol, 2 eq) was dissolved in dimethylformamide (25 mL) and added to the previous solution, followed by *O*-(7-azabenzotriazol-1-yl)-*N,N,N',N'*-tetramethyluronium hexafluorophosphate (1.08 g, 2.84 mmol, 1.9 eq) in dichloromethane (5 mL). *N,N'*-diisopropylethylamine (0.784 mL, 4.5 mmol, 3 eq) was added and the reaction mixture was stirred for 2h at +4°C. When mitomycin C was totally converted, dichloromethane (300 mL) was added to the reaction. The mixture was washed twice with a solution of NaCl 1M (100 mL), and once with a solution of aqueous citric acid 5% (100 mL). Residual water was removed by treatment with Na₂SO₄ and the organic phase was filtered and concentrated using a rotavapor. To the residual dimethylformamide of the concentrated organic layer was added dichloromethane (40 mL) and the mixture was precipitated using *tert*-butyl methyl ether (MTBE, 200 mL) at +4°C under agitation. The precipitate was filtered and dried. The purple precipitate was solubilised in a mixture of dichloromethane / methanol (98/2 v/v). This solution was purified by flash-chromatography on silica (dichloromethane 100% v/v to dichloromethane/methanol 96/4 v/v). The recovered fractions were pooled and concentrated by rotary evaporation.
Purity: 94% (determined by HPLC at 214 nm).
MS (ES⁺): 925 [MH]⁺

### I.4.2 Synthesis of ALAL-mitomycin C

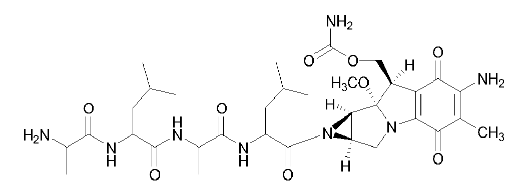

Fmoc-ALAL-mitomycin C (1 g, 1.08 mmol, 1 eq) was dissolved in dimethylformamide (22 mL) and piperidine (5.34 mL, 54 mmol, 50 eq) was added. The solution was stirred 90 seconds at room temperature and directly neutralized at +4°C with a cold solution of HC1 1N (9.0 mL, 54 mmol, 50 eq to obtain a pH of 7.4). The solution was stirred 10 min at +4°C and precipitated with MTBE (450 mL) at +4°C. The precipitate was dissolved in water (400 mL) and the pH of the aqueous layer was adjusted at 8.8-9.0 with a solution of NaOH 1M. Then ALAL-mitomycin C was extracted 5 times with dichloromethane (300 mL). The organic layers were pooled, dried on Na₂SO₄, filtered and concentrated by rotary evaporation to obtain a purple solid.
Purity: 94% (determined by HPLC at 214 nm).
MS (ES⁺): 702 [MH]⁺

### I.4.3 Synthesis of TetraAcid-ALAL-mitomycin C

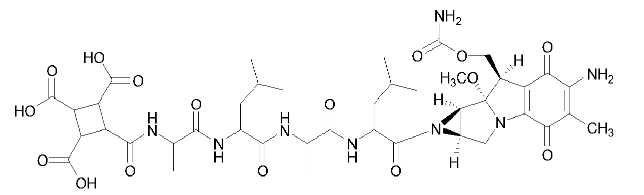

ALAL-mitomycin C (0.4 g, 0.57 mmol, 1 eq) was dissolved in dimethylformamide (35 mL) at +4°C. *N,N'*-diisopropylethylamine (497 µL, 2.85 mmol, 5 eq) was added followed by 1,2,3,4-cyclobutanetetracarboxylic acid (1.057 g, 4.55 mmol, 8 eq). Finally *O*-(7-azabenzotriazol-1-yl)-*N,N,N',N'*-tetramethyluronium hexafluorophosphate (0.323 g, 0.85 mmol, 1.5 eq) was added and the reaction mixture was stirred at +4°C for 45 minutes. When ALAL-mitomycin C was totally converted, the reaction mixture was precipitated using *tert*-butyl methyl ether (700 mL) at +4°C. After filtration and drying, the precipitate was dissolved in a solution of NaOH 1M and then purified by preparative HPLC. The recovered fractions were pooled and the solution was lyophilized to obtain a purple solid.
Purity: 90.6% (determined by HPLC at 214 nm).
MS (ES⁺): 939 [M+Na]⁺

### I.5 Method of synthesis of Suc-βALAL-mitomycin C

### I.5.1 Synthesis of MeO-Suc-βALAL-mitomycin C

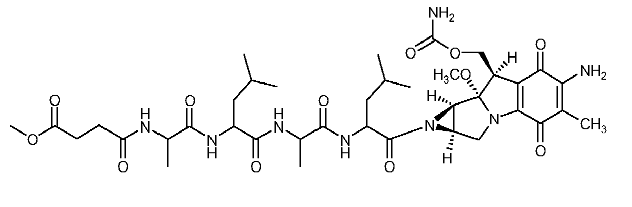

Mitomycin C (MMC, 0.400 g, 1.20 mmol, 1 eq) and MeO-Suc-βALAL-OH (1.200 g, 2.40 mmol, 2 eq) were dissolved in dimethylformamide (15 mL) at +4°C. *N,N'-*diisopropylethylamine (417 µL, 2.40 mmol, 2 eq) was added and the reaction mixture was stirred for 10 minutes at +4°C. A solution of 2-(6-chloro-1H-benzotriazole-1-yl)-1,1,3,3-tetramethyluronium hexafluorophosphate (0.57 g, 1.38 mmol, 1.15 eq) in dimethylformamide (5 mL) was added dropwise and the reaction mixture was kept at +4°C. The reaction mixture was stirred at +4°C for 3 hours. Dichloromethane (60 mL) was added to the reaction. The organic layer was washed with a solution of NaCl 1M (35 mL) 4 times. The organic layer was concentrated using a rotavapor. Dichloromethane (10 mL) was added to the residual dimethylformamide of the concentrated organic layer and the solution was precipitated using *tert*-butyl methyl ether (100 mL) at +4°C. The precipitate was filtered and dried. The resulting solid was dissolved in a mixture of dichloromethane / methanol (95/5 v/v). The solution was purified by flash-chromatography (dichloromethane 100% v/v to dichloromethane/methanol 90/10 v/v). The recovered fractions were pooled and concentrated by rotary evaporation.
Purity: 95.4% (determined by HPLC at 214 nm).
MS (ES⁺): 839 [M+Na]⁺

### I.5.2 Synthesis of Suc-βALAL-mitomycin C

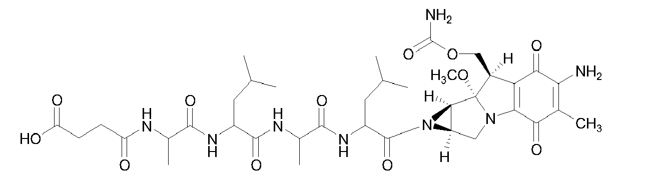

*Candida antarctica* lipase B cross-linked enzyme aggregates (CALB-CLEA, CLEA Technologies) (0.2 g) was washed 3 times with water (5 mL), and added in water (5 mL) to MeO-Suc-βALAL-mitomycin C. This mixture was stirred at +37°C overnight and filtered. The aqueous filtrate was purified by preparative HPLC. The recovered fractions were pooled and lyophilized.
Purity: 94 % (determined by HPLC at 214 nm).
MS (ES⁺): 825 [M+Na]⁺

### I.6 Method of synthesis of TetraAcid-ALAL-paclitaxel

### I.6.1 Synthesis of Fmoc-ALAL-paclitaxel

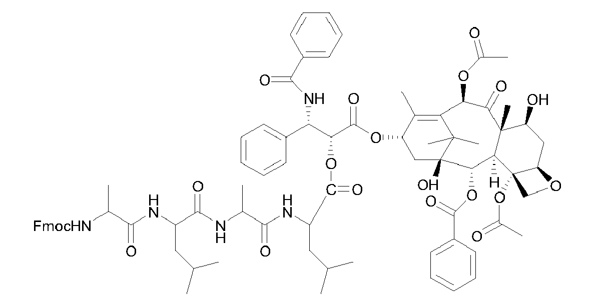

Paclitaxel (0.214 g, 0.251 mmol, 1 eq) was dissolved in dichloromethane (14 mL) at +4°C. 4-di(methylamino)pyridine (0.184 g, 1.504 mmol, 6 eq) was added, followed by a solution of Fmoc-ALAL-OH (0.460 g, 0.752 mmol, 3 eq) in dimethylformamide (4 mL). The solution was stirred at +4°C for 10 minutes then *N*-(3-dimethylaminopropyl)-*N'-*ethylcarbodiimide hydrochloride (0.145 g, 0.752 mmol, 3 eq) was added. The solution was stirred at +4°C for 10 minutes, then 3 hours at room temperature. Dichloromethane was added (100 mL) and the solution was washed twice with NaCl 1M (50 mL) and twice with aqueous citric acid 5% (50 mL). The organic layer was concentrated using a rotavapor, dissolved in a minimum of dichloromethane and precipitated using *tert*-butyl methyl ether at +4°C. The pale yellow solid was filtered and dried.
Purity: 90%.
MS (ES⁺): 1444 [MH]⁺

### I.6.2 Synthesis of ALAL-paclitaxel

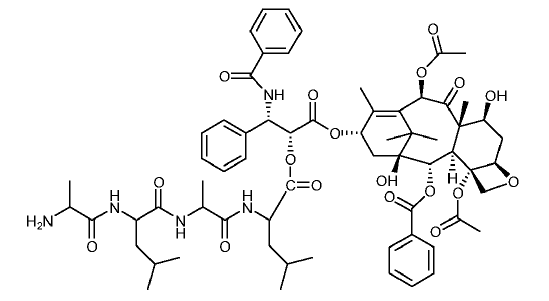

The total quantity of Fmoc-ALAL-paclitaxel for the initial stage of the synthesis was dissolved in dimethylformamide (5 mL), and piperidine (1.25 mL, 50 eq) was added. The solution was stirred at room temperature for 90 seconds and directly quenched at +4°C with HC16N (2.10 mL, 50 eq).

Dichloromethane (50 mL) was added and the solution was washed three times with H₂O (50 mL) and once with NaCl 1M (50 mL). The organic layer was evaporated to obtain a pale yellow solid.
Purity: 90%.
MS (ES⁺): 1222 [MH]⁺

### I.6.6 Synthesis of TetraAcid-ALAL-paclitaxel

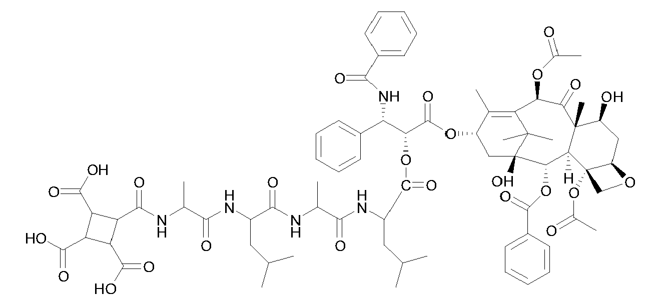

The total quantity of ALAL-paclitaxel generated in the previous step was dissolved in dimethylformamide (8 mL) and *N*,*N*'-diisopropylethylamine (0.13 mL, 0.070 mmol, 3 eq) was added. The solution was stirred 5 minutes at room temperature then 1,2,3,4-cyclobutanetetracarboxylic acid (0.580 g, 2.510 mmol, 10 eq) was added followed by 2-(6-chloro-1H-benzotriazole-1-yl)-1,1,3,3-tetramethyluronium hexafluorophosphate (0.155 g, 0.377 mmol, 1.5 eq). When ALAL-paclitaxel was totally converted, the crude conjugate was dissolved in phosphate buffer (pH 7.1, 1M) (3 mL) and a sufficient volume of acetonitrile was added to maintain a clear solution. The solution was purified by preparative HPLC. The recovered HPLC fractions were pooled and lyophilized.
Purity: 99.2% (determined by HPLC at 214 nm).
MS (ES⁺): 1434 [MH]⁺

### I.7 Method of synthesis of Suc-βALAL-paclitaxel

### I.7.1 Synthesis of tBu-SucβALAL-paclitaxel

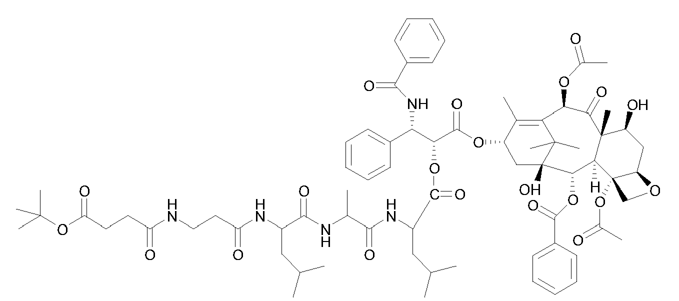

Paclitaxel (20 mg, 0.023 mmol, 1 eq) was dissolved in dichloromethane (0.5 mL) at +4°C. To this solution was added 4-di(methylamino)pyridine (17 mg, 0.141 mmol, 6 eq). tBu-Suc-βALAL-OH (38 mg, 0.070 mmol, 3 eq) was added at +4°C followed by N-(3-dimethylaminopropyl)-*N'*-ethylcarbodiimide hydrochloride (13 mg, 0.070 mmol, 3 eq). The solution was stirred at +4°C for 10 minutes, then 30 minutes at room temperature. Dichloromethane (5 mL) was added and the organic layer was washed twice with citric acid 5% (2 mL), twice with NaHCO₃ 1M (2 mL) and twice with NaCl 1M (2 mL). The organic layer was evaporated using a rotavapor until only a residual amount of solvent remained. The tBu-Suc-βALAL-paclitaxel was precipitated using *tert*-butyl methyl ether (5 mL).
MS (ES⁺): 1378 [MH]⁺

### I.7.2 Synthesis of SucβALAL-paclitaxel

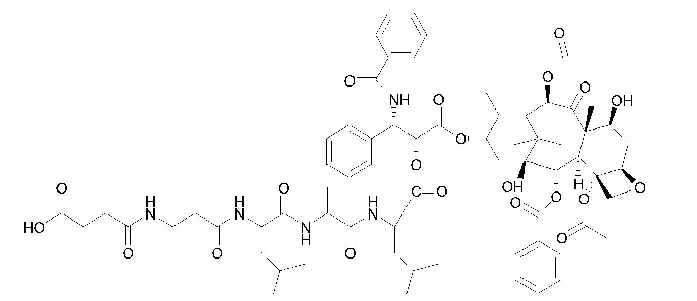

tBu-Suc-βALAL-paclitaxel (5 mg, 0.036 mmol, 1eq) was dissolved in a solution of trifluoroacetic acid/dichloromethane/triisopropylsilane (100µL/200µL/5µL) and stirred for 20 minutes at room temperature. The solution was neutralized (pH 6) with *N,N'-*diisopropylethylamine. The crude conjugate was purified by preparative HPLC and the recovered fractions were pooled and lyophilized.
Purity: 93% (determined by HPLC at 214 nm).
MS (ES⁺): 1344 [M+Na]⁺

### II. In vitro human plasma stability studies of TA-ALAL-Drug conjugates

### II.1 Method

Human plasma (citrated human blood was centrifuged at 600 g for 10 min at +4°C, plasma was recovered and aliquots stored at -20°C) was used to assess the stability of the following drug conjugates: Suc-ALAL-doxorubicin, Suc-βALAL-doxorubicin, TA-ALAL-doxorubicin, Suc-βALAL-MMC, TA-ALAL-MMC, Suc-βALAL-paclitaxel and TA-ALAL-paclitaxel.
The drug conjugates (50 µM doxorubicin or MMC, 100 µM paclitaxel conjugates) were incubated at 37°C with human plasma either for 0, 2 or 6 hours, 50 µL of sample were collected at each time point and extraction was performed immediately.

For the doxorubicin conjugate and metabolites, 150 µL of acetonitrile was added to the 50 µL samples. Samples were vortexed and centrifuged for 10 min at 13 000 rpm at room temperature. 100 µL were harvested and 200 µL of 2% ammonium formate pH 4.0 were added before centrifugation (10 min at 13 000 rpm). HPLC analysis (at 560 nm) was performed using the supernatant.

For MMC conjugate and metabolites were extracted prior to HPLC analysis. 100 µL of acetonitrile was added to the 50 µL of sample, prior to vortexing (10 seconds) and centrifugation (10 min at 13 000 rpm) at room temperature. 50 µL of supernatant was recovered and mixed with 150 µL of Phosphate Buffer Saline (PBS) 1X. After centrifugation (10 min at 13 000 rpm), 150 µL of supernatant were collected for HPLC analysis. Stability and reactivation were evaluated by HPLC analysis (at 270 nm) and MMC conjugate and metabolite peak areas were compared.

For the paclitaxel conjugate and metabolites, 150 µL of acetonitrile was added to 50 µL of sample, vortexed and centrifuged for 10 min at 13 000 rpm at room temperature. 100 µL of supernatant was recovered and mixed with 100 µL of water. After centrifugation (10 min at 13 000 rpm), the supernatant was collected for HPLC analysis (at 230 nm).

### II.2 Results

All three conjugates Suc-ALAL-doxorubicin, Suc-βALAL-doxorubicin and TA-ALAL-doxorubicin were stable in human plasma after 2 hours incubation and no difference in stability between the three conjugates could be detected: the percentage of intact conjugate observed after 2 hours in human plasma was 80, 77.2 and 83% respectively, whilst after 6 hours the percentage of intact conjugate was 54, 47.5 and 67% respectively. For Suc-βALAL-MMC and TA-ALAL-MMC conjugates no difference in plasma stability was observed with 73 and 78% of conjugates, respectively remaining after 6 hours.
The plasma stability of Suc-βALAL-paclitaxel and TA-ALAL-paclitaxel conjugates was also similar (41 and 43% respectively after 6 hours).

### III In vitro evaluation of cytotoxic activity of TA-ALAL-drug conjugates

### III.1 Method

The cytotoxicity of different drug conjugates was evaluated using the HCT 116 colon cancer cell line and was compared to the cytotoxicity of the free drug (doxorubicin, mitomycin C or paclitaxel). Cells were seeded at 8 000 cells/well and maintained in Mc Coy medium supplemented with 10% fetal bovine serum for 24 hours before treatment. Cells were incubated with increasing concentrations of drugs or conjugates at 37°C, 5% CO₂. The medium was then removed and a WST-1 test (purchased from Roche) was performed according to the manufacturer's instructions.
The cytotoxicity of the tested conjugates was expressed as the molar concentration inhibiting 50% of the cell viability (IC₅₀), calculated using Graph Pad Prism 3.02 software. Experimental data are presented as the mean of three independent experiments performed in duplicate.

### III.2 Results

The intact prodrug should be inactive in comparison with the free drug, in order to allow the prodrug to circulate in an inactive, non-toxic, form and should only be reactivated by tumor specific endopeptidases in the tumor environment. In vitro experiments were performed in the HCT116 cell line to confirm that the prodrug conjugate is inactive. HCT116 cells do not secrete sufficient endopeptidases into the medium to allow activation of the prodrug, under the conditions used. The cytotoxicity of the different drug conjugates are shown in Table 1 below. All drug conjugates (TA-ALAL-drug, Suc-ALAL-drug or Suc-βALAL-drug) showed a significant level of inactivation compared to the non-conjugated drug. This data confirms that conjugation of TA-ALAL to the cytotoxic drugs tested (e.g. mitomycin C, doxorubicin or paclitaxel) results in inactivation of the cytotoxic drug, presumably through inhibition of the internalisation of the cytotoxic molecule and therefore its interaction with its therapeutic target.

**Table 1: Drug concentration that inhibits 50% of cell viability (IC₅₀) using the HCT 116 cell line. Data represent the mean value of 3 independent experiments. After the indicated exposure time at 37°C, cells were rinsed and a viability assay (WST1 test) was performed to determine IC₅₀.**

| Drugs or drug conjugates | IC₅₀ values | Incubation time |
|---|---|---|
| doxorubicin (dox) | 0.034 µM | 48 h |
| TA-ALAL-dox | > 10 µM | 48 h |
| Suc-βALAL-dox | > 10 µM | 48 h |
| Suc-ALAL-dox | 10 µM | 48 h |
| Mitomycin C (MMC) | 5 µM | 24 h |
| TA-ALAL-MMC | 86 µM | 24 h |
| Suc-βALAL-MMC | 67 µM | 24 h |
| paclitaxel | 0.020 µM | 4h + 72 h |
| TA-ALAL-paclitaxel | 10 µM | 4h + 72 h |
| Suc-βALAL-paclitaxel | 762 nM | 4h + 72 h |

### IV Evaluation of the in vivo toxicity of TA-ALAL-doxorubicin conjugate compared to known doxorubicin conjugates

Toxicity studies were conducted in LS 174T tumor-bearing nude mice following intravenous administration. This model was used to assess the toxicity of TA-ALAL-doxorubicin as this model has been shown to over-express CD10 as well as TOP. This model has also been shown to be responsive to Suc-βALAL-doxorubicin (Dubois et al., Cancer Research, 62, 2327-2331 (2002)).
LS 174T tumors were established by a subcutaneous implantation of cells (3 x 10⁷ cells injected) in the right flank of 7 weeks old female NMRI nude mice (Janvier, France). The day of the first injection, animals were randomly assigned to groups of 6 animals. The TA-ALAL-doxorubicin and Suc-ALAL-doxorubicin conjugates were dissolved in saline and Suc-βALAL-doxorubicin was prepared in glucose 5%. All conjugates were delivered by bolus intravenous injection (i.v.) in the lateral tail vein at 10 µL/g. During the course of the experiment, clinical signs and body weight were controlled at least twice a week. The experiment was repeated twice. Results are shown in Table 2 below.

**Table 2: In vivo toxicity of TA-ALAL-doxorubicin conjugate compared to Suc-βALAL-doxorubicin and Suc-ALAL-doxorubicin in LS 174T tumor-bearing mice.**

| | Doxorubicin conjugates | Dose (µmol/kg) | Maximal body weight loss % |
|---|---|---|---|
| Experiment 1 | TA-ALAL-dox | 67.7 | 13.7 |
| | Suc-βALAL-dox | 67.7 | 19.9 |
| | Suc-ALAL-dox | 67.7 | 36.1 (3 dead) |
| Experiment 2 | TA-ALAL-dox | 55.1 | 11.3 |
| | | 77.4 | 17.9 |
| | Suc-βALAL-dox | 33.8 | 10.6 |
| | | 47.4 | 15.9 |

At equivalent doses (experiment 1, Table 2) TA-ALAL-dox induced less body weight loss than Suc-βALAL-dox, suggesting that TA-ALAL-dox has a better blood stability than Suc-βALAL-dox. On the contrary replacement of the βAla by an Ala in the conjugate induced a loss of *in vivo* stability as an equivalent dose of Suc-ALAL-dox produced an important body weight loss and the death of 3 animals in the group. Experiment 2 (Table 2) shows the doses of TA-ALAL-dox and Suc-βALAL-dox required to have an equitoxic effect. This experiment is consistent with experiment 1 (Table 1), confirming that TA-ALAL-dox is less toxic than Suc-βALAL-dox and shows that equitoxic dose of TA-ALAL-dox and Suc-βALAL-dox are 77.4 and 47.4 µmol/kg respectively.

### V Evaluation of the in vivo efficacy of TA-ALAL-doxorubicin conjugate compared to known doxorubicin conjugates in the LS 174T tumor model

LS 174T tumors were established by a subcutaneous implantation of cells (3 x 10⁷ cells injected) in the right flank of 7 weeks old female NMRI nude mice (Janvier, France). Treatments were initiated when the tumors had reached a size of 100 mm³ (calculated using the following formula: [length x width²]/2). The day of the first injection animals were randomly assigned to groups of 6 animals. The TA-ALAL-doxorubicin and Suc-ALAL-doxorubicin conjugates were dissolved in saline and Suc-βALAL-doxorubicin conjugate was prepared in glucose 5%. The conjugates were delivered by bolus intravenous injection (i.v.) in the lateral tail vein at 10 µL/g. During the course of the experiment, clinical signs, body weight and tumor volume were controlled twice a week. Results are presented as the evolution of mean tumor volume as a function of time. Optimal T/C (ratio of mean tumor volume of treated *versus* control mice) values were used as a measure of treatment efficacy. The optimal T/C% reflects the maximal tumor growth inhibition achieved. The experiment was repeated twice. Results are shown in Table 3 below.

**Table 3: Anti-tumoral efficacy of TA-ALAL-doxorubicin compared to Suc-βALAL-doxorubicin and Suc-ALAL-doxorubicin in the LS 174T tumor model.**

| | Doxorubicin conjugates | Dose (µmol/kg) | Optimal T/C (%) |
|---|---|---|---|
| Experiment 1 | TA-ALAL-dox | 67.7 | 42 |
| | Suc-βALAL-dox | 67.7 | 52 |
| | Suc-ALAL-dox | 24.2* | 70 |
| | Suc-ALAL-dox | 67.7 | 3 animals died |
| Experiment 2 | TA-ALAL-dox | 77.4 | 36 |
| | Suc-βALAL-dox | 47.4 | 52 |

| | | | |
|---|---|---|---|
| * Higher doses were toxic. | | | |

In these experiments, TA-ALAL-dox shows an increased antitumoral efficacy as compared to both suc-βALAL-dox and suc-ALAL-dox.

### VI Evaluation of the in vivo efficacy of TA-ALAL-mitomycin C conjugate in the LS 174T tumor model

The *in vivo* anti-tumoral efficacy of TA-ALAL-MMC was compared to that of Suc-βALAL-MMC in the LS 174T tumor model. The establishment of the LS 174T tumors and the treatments were carried out as previously described in Example V. TA-ALAL-MMC conjugate was dissolved in saline and Suc-βALAL-MMC conjugate was prepared in 3% DMSO in Sodium phosphate buffer (15mM). The conjugates were delivered by bolus intravenous injection (i.v.) in the lateral tail vein at 10 µL/g. During the course of the experiment, clinical signs, body weight and tumor volume were controlled twice a week. The experiment was repeated twice. Results are shown in Table 4 below.

**Table 4: Anti-tumoral efficacy of TA-ALAL-MMC compared to Suc-βALAL-MMC in the LS 174T tumor model.**

| Conjugates | Dose (µmol/kg) | Optimal T/C (%) | Maximal body weight loss % |
|---|---|---|---|
| Experiment 1 | | | |
| TA-ALAL-MMC | 60 | 28 | 16.2 |
| Suc-βALAL-MMC | 60 | 48 | 14.5 |
| Experiment 2 | | | |
| TA-ALAL-MMC | 60 | 19 | 19 |
| Suc-βALAL-MMC | 80 | 43 | 15.9 |
| Suc-ALAL-MMC | 40 | 52 | 13.7 |

| | | | |
|---|---|---|---|
| TA-ALAL-MMC showed an increased antitumoral efficacy as compared to Suc-βALAL-MMC. | | | |

## Claims

1. A compound having the following formula : 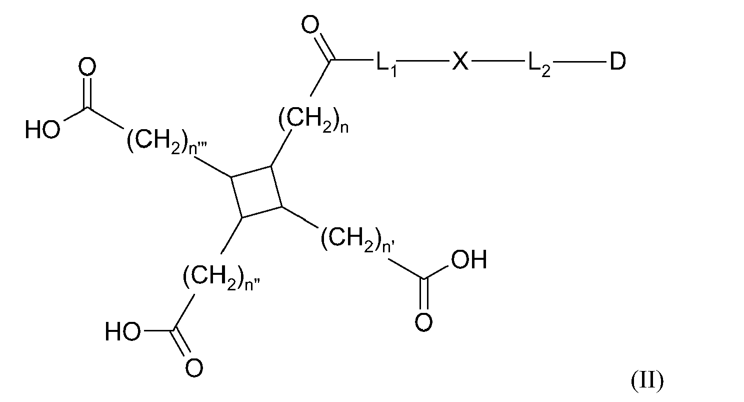 wherein
L₁ and L₂ are independently a covalent bond or a linking moiety,
n, n', n" and n"' independently represent an integer comprised between 0 and 10,
X is a cleavable oligopeptide moiety, and
D is a therapeutic agent or marker,
or the pharmaceutically acceptable salts thereof.

2. The compound according to claim 1, wherein said compound is represented by formula (III): 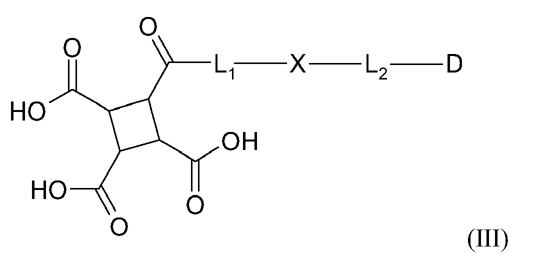 or the pharmaceutically acceptable salts thereof.

3. The compound according to claim 1 or 2, wherein said cleavable oligopeptide moiety comprises 2 to 10 amino acid residues, preferably 3 to 7 amino acid residues.

4. The compound of claim 1 or 2, wherein the oligopeptide comprises one or more of the following amino acid pairings: Arg-Leu, Arg-Phe, Arg-Val, Ala-Phe, Ala-Leu, Ala-Tyr, Cys-Arg, Cys-Asp, Cys-Phe, Gln-Phe, Gly-Asp, Gly-Phe, Gly-Leu, Gly-Gln, Gly-Gly, Gly-Pro, His-Ser, Ile-Ala, Leu-Gln, Leu-Gly, Leu-Leu, Leu-Phe, Leu-Tyr, Lys-Leu, Met-Leu, Pro-Phe, Pro-Tyr, Pro-Leu, Phe-Leu, Phe-Phe, Tyr-Ile, Tyr-Pro, Tyr-Leu, Val-Tyr, Val-Phe, Ser-Leu, and Ser-Lys.

5. The compound of claim 4, wherein the oligopeptide comprises one or more of the following sequences: (Leu)y-(Ala-Leu)x-Ala-Leu or (Leu)y-(Ala-Leu)x-Ala-Phe wherein y = 0 or 1 and x = 1, 2, or 3.

6. The compound of claim 1 or 2, wherein the oligopeptide comprises one or more of the following sequences: Ala-Phe-Lys, Ala-Leu-Ala-Leu (SEQ ID No. 1), beta-Ala-Leu-Ala-Leu (SEQ ID No. 2), Ala-Leu-Lys-Leu-Leu (SEQ ID No. 3), Ala-Tyr-Gly-Gly-Phe-Leu (SEQ ID No. 4), His-Ser-Ser-Lys-Leu-Gln-Leu (SEQ ID No. 5), Gly-Pro-Leu-Gly-Ile-Ala-Gly-Gln (SEQ ID No. 6) and Cys-Asp-Cys-Arg-Gly-Asp-Cys-Phe-Cys (SEQ ID No. 7).

7. A compound according to claim 1 or 2, wherein said cleavable oligopeptide moiety comprises the following amino acid sequence: Ala-Leu-Ala-Leu.

8. A compound according to claim 1 or 2, wherein the oligopeptide is cleavable by at least one enzyme that is present in the environment of one or more cells that are selected from the group of: tumor cells, stromal cells of tumors, neoangiogenic endothelial cells of tumors and tumor metastases, macrophages, monocytes, polymorphonuclear leukocytes or lymphocytes that infiltrate tumors and tumor metastases.

9. A compound according to claim 8, wherein the enzyme is a peptidase selected from the group of: neprilysin (CD10), thimet oligopeptidase (TOP), prostate specific antigen (PSA), plasmin, legumain, collagenases, urokinase, cathepsins, human fibroblast activation protein (FAPα) or the matrix metallopeptidases.

10. A compound according to any of the preceding claims, wherein the therapeutic agent is selected from the following group: a chemical agent, a polypeptide, a protein, an antibody a nucleic acid, an antibiotic, or a virus.

11. A compound according to claim 10, wherein the therapeutic agent has anti-tumor therapeutic activity, anti-angiogenic activity or anti-inflammatory activity.

12. A compound according to claim 10, wherein the therapeutic agent is selected from the group of: vincristine, vinblastine, vindesine, vinorelbine, paclitaxel, docetaxel, 10-deacetyltaxol, 7-*epi*-taxol, baccatin III, 7-xylosyltaxol, isotaxel, ifosfamide, melphalan, chloroaminophene, procarbazine, chlorambucil, thiophosphoramide, busulfan, dacarbazine (DTIC), mitomycins, nitroso-ureas, estramustine, BCNU, CCNU, fotemustine, streptonigrin, cisplatin, carboplatin, oxaliplatin, methotrexate, aminopterin, 5-fluorouracil, 6-mercaptopurine, raltitrexed, cytosine arabinoside, adenosine arabinoside, gemcitabine, cladribine, pentostatin, fludarabine phosphate, hydroxyureas, camptothecin, irinotecan, topotecan, 9-dimethylaminomethyl-hydroxy-camptothecin hydrochloride, etoposide, teniposide, amsacrine; mitoxantrone; L-canavanine, doxorubicin, THP-adriamycin, daunorubicin, idarubicin, rubidazone, pirarubicin, zorubicin, aclarubicin, epiadriamycin (4'epi-adriamycin or epirubicin), mitoxantrone, bleomycins, actinomycins including actinomycin D, streptozotocin, calicheamycin, duocarmycins, combretastatin; L-asparaginase; hormones; pure inhibitors of aromatase; androgens, analogs-antagonists of LH-RH; interferon alpha (IFN-alpha), interferon gamma (IFN-gamma), interleukin 1 (IL-1), IL-2, IL-4, IL-6, IL-10, IL-12, IL-15, tumor necrosis factor-alpha (TNF-alpha), IGF-1 antagonists (insulin-like growth factor); proteasome inhibitors; farnesyl-transferase inhibitors (FTI); epothilones; maytansinoids; discodermolide; fostriecin; inhibitors of tyrosine kinases such as STI 571 (imatinib mesylate); receptor tyrosine kinase inhibitors such as erlotinib, sorafenib, vandetanib, canertinib, PKI166, gefitinib, sunitinib, lapatinib, EKB-569; Bcr-Ab1 kinase inhibitors such as dasatinib, nilotinib, imatinib; aurora kinase inhibitors such as VX-680, CYC116, PHA-739358, SU-6668, JNJ-7706621, MLN8054, AZD-1152, PHA-680632; CDK inhibitors such as flavopirodol, seliciclib, E7070, BMS-387032; MEK inhibitors such as PD184352, U-0126; mTOR inhibitors such as CCI-779 or AP23573; Kinesin spindle inhibitors such as; SB 743921 or MK-0731; RAF/MEK inhibitors such as; Sorafenib, CHIR-265, PLX-4032, CI-1040, PD0325901 or ARRY-142886; bryostatin; L-779450; LY333531; endostatins; proteins; peptides; antibodies; and anti-inflammatory cytokines.

13. A compound according to any of the preceding claims, wherein the linking moities L₁ or L₂ are selected from the group comprising bi- and multivalent organic radicals independently selected from substituted or unsubstituted alkyl, substituted or unsubstituted heteroalkyl and substituted or unsubstituted aryl, aldehydes, acids, esters and anhydrides, ketone, acyl halide, quinone, alkylene, cycloalkyl, heterocycloalkyl, sulphydryl or carboxyl groups, such as maleimido acid derivatives, and succinimido derivatives or may be derived from, succinimidyl esters.

14. A compound according to claim 13, wherein the linking moiety L₂ is selected from the group comprising *N,N'*-ethylenediamine, ethanolamine, aminocaproic acid, 4-aminobenzyl alcohol, 6-Amino-1-hexanol, 1,3-diaminopropane, cysteamine, 1,4-diaminobutane, *N*-methyl-1,3-propanediamine, *N*-methylethylenediamine and 4-aminobenzylamine.

15. A pharmaceutical composition comprising in a pharmaceutically acceptable carrier at least one compound as defined in any one of the claims 1-14.

16. The pharmaceutical composition according to claim 15, intended for an intra-cranial, intra-spinal, enteral or parenteral administration.

17. The pharmaceutical composition according to claim 14 or 15, for the treatment of cancers.

18. The pharmaceutical composition according to claim 17, for the treatment of breast cancers, colorectal cancers, liver cancers, lung cancers such as small cell, non-small cell, bronchic cancers, prostate cancers, ovarian cancers, brain cancers, and pancreatic cancers, colon cancers, head and neck cancers, stomach cancers, bladder cancers, non-Hodgkin's lymphomas, melanomas, leukaemias, neuroblastomas, or glioblastomas.

19. A use of an effective amount of at least one compound as defined by one of claims 1 to 14 for the preparation of a pharmaceutical composition for the treatment of cancers or infectious diseases.

20. A pharmaceutically acceptable salt of a compound according to claims 1 to 14.
